(19) Europäisches Patentamt

European Patent Office

Office européen des brevets

(11) **EP 1 213 685 B1**

(12) **EUROPÄISCHE PATENTSCHRIFT**

(45) Veröffentlichungstag und Bekanntmachung des Hinweises auf die Patenterteilung:
**31.03.2004 Patentblatt 2004/14**

(51) Int Cl.⁷: **G06T 11/00**

(21) Anmeldenummer: **01000712.8**

(22) Anmeldetag: **05.12.2001**

(54) **Computertomographie-Verfahren mit helixförmiger Relativbewegung**

Method of computed tomography with helical relative movement

Procédé de tomographie par ordinateur avec balayage helicoidal

(84) Benannte Vertragsstaaten:
**DE FR GB NL**

(30) Priorität: **07.12.2000 DE 10061120**

(43) Veröffentlichungstag der Anmeldung:
**12.06.2002 Patentblatt 2002/24**

(73) Patentinhaber:
• **Philips Intellectual Property & Standards GmbH**
**20099 Hamburg (DE)**
Benannte Vertragsstaaten:
**DE**
• **Koninklijke Philips Electronics N.V.**
**5621 BA Eindhoven (NL)**
Benannte Vertragsstaaten:
**DE FR GB NL**

(72) Erfinder:
• **Danielsson, Per-Erik**
**52066 Aachen (DE)**
• **Turbell, Henrik**
**52066 Aachen (DE)**

(74) Vertreter: **Volmer, Georg, Dipl.-Ing. et al**
**Philips Intellectual Property & Standards GmbH,**
**Postfach 50 04 42**
**52088 Aachen (DE)**

(56) Entgegenhaltungen:
**US-A- 5 802 134         US-A- 6 151 377**

• **H. TURBELL, P.-E. DANIELSSON: "The PI-FAST Method for Approximate Helical Cone-Beam Reconstruction" SIXTH INTERNATIONAL CONFERENCE ON FULLY THREE-DIMENSIONAL IMAGE RECONSTRUCTION IN RADIOLOGY AND NUCLEAR MEDICINE, [Online] 31. Oktober 2001 (2001-10-31), XP002192061 Pacific Grove, California, USA Gefunden im Internet: <URL:http://cfi.lbl.gov/3D-2001/abstracts/ 05-3.pdf> [gefunden am 2002-03-04]**
• **TURBELL H ET AL: "HELICAL CONE-BEAM TOMOGRAPHY" INTERNATIONAL JOURNAL OF IMAGING SYSTEMS AND TECHNOLOGY, WILEY AND SONS, NEW YORK, US, Bd. 11, Nr. 1, 2000, Seiten 91-100, XP000963723 ISSN: 0899-9457**
• **DANIELSSON P-E ET AL: "Backprojection in O(N2logN) time " NUCLEAR SCIENCE SYMPOSIUM, 1997. IEEE ALBUQUERQUE, NM, USA 9-15 NOV. 1997, NEW YORK, NY, USA,IEEE, US, 9. November 1997 (1997-11-09), Seiten 1279-1283, XP010275650 ISBN: 0-7803-4258-5**

**Beschreibung**

Die Erfindung betrifft ein Computertomographie Verfahren, bei dem eine ein kegelförmiges Strahlenbündel emittierende Strahlenquelle relativ zu einem Untersuchungsbereich eine Relativbewegung in Form einer Helix ausführt. Außerdem betrifft die Erfindung einen Computertomographen und ein Computerprogramm zur Durchführung dieses Verfahrens

Ein solches Computertomographie-Verfahren ist aus der PCT/IB 99/00027 (PHQ 98-020) bekannt. Die Rekonstruktion eines CT-Bildes aus den akquirierten Meßwerten erfolgt dabei nach Methode der gefilterten Rückprojektion, wobei zunächst die Meßwerte nach einem sogenannten parallelen Rebinning einer eindimensionalen rampenförmigen Filterung unterzogen werden. Die Filterdaten, die zu Strahlen gehören, die einen bestimmten Punkt im Untersuchungsbereich durchsetzt haben, werden anschließend summiert, woraus sich der Schwächungskoeffizient für die Strahlen des Strahlenbündels in dem betreffenden Punkt ergibt.

Trotz einer ansprechenden Qualität des auf diese Weise erzeugten CT-Bildes (CT = Computertomographie) können sich auch darin noch Bildartefakte zeigen, insbesondere wenn das kegelförmige Strahlenbündel senkrecht zur Rotationsachse und parallel dazu einen großen Öffnungswinkel hat.

Es ist Aufgabe der vorliegenden Erfindung die Bildqualität bei einem Verfahren der eingangs genannten Art noch weiter zu verbessern. Diese Aufgabe wird gelöst durch ein Computertomographie-Verfahren mit den Schritten

a) Erzeugen eines kegelförmigen Strahlenbündels, das eine Vielzahl von Strahlen enthält, die von einer Strahlenquelle ausgehen und einen Untersuchungsbereich bzw. ein darin befindliches Objekt durchsetzen,

b) Erzeugung einer Relativbewegung zwischen der Strahlenquelle einerseits und dem Untersuchungsbereich bzw. dem Objekt andrerseits, die eine Rotation um eine Rotationsachse und eine Verschiebung parallel zur Rotationsachse umfaßt und die Form einer Helix hat,

c) Akquisition von Meßwerten, die von der Schwächung der Strahlen in dem Untersuchungsbereich abhängen, mit einer Detektoreinheit während der Relativbewegung,

d) Berechnung von Verbindungswerten durch Summation der Meßwerte entlang von Verbindungslinien eines Netzes in einem dreidimensionalen, die Lage und die Orientierung der Strahlen beschreibenden Parameterraum

e) Filtern der Verbindungswerte zur Erzeugung von Filterdaten für Verbindungslinien, die Strahlen zugeordnet sind, welche eine bestimmte Fläche des Untersuchungsbereiches durchsetzen,

f) Berechnung der Schwächung der Strahlung in Pixeln auf der Fläche durch Summation der Filterdaten von Verbindungslinien, die die Trajektorie approximieren, die in dem Parameterraum durch die Strahlen definiert wird, die das jeweilige Pixel durchsetzen,

g) Wiederholung zumindest der Schritte e) und f) für andere Flächen, die in Richtung der Rotationsachse gegeneinander versetzt sind.

Die Erfindung basiert auf der Erkenntnis, daß die bei den bekannten Verfahren auftretenden Artefakte dadurch hervorgerufen werden, daß sich die Zusammensetzung der Meßwerte, die einer gemeinsamen (eindimensionalen und rampenförmigen) Filteroperation unterzogen werden, sich von Filteroperation zu Filteroperation ändert. Bei der Erfindung werden für die verschiedenen Filteroperationen hingegen nur Meßwerte herangezogen, die denjenigen Strahlen zugeordnet sind, die - zumindest näherungsweise - ein und dieselbe Fläche im Untersuchungsbereich durchsetzen.

Ermöglicht wird die Beschränkung der Filteroperationen auf diese Meßwerte dadurch, daß eine zweistufige Summation gemäß den Merkmalen d und f erfolgt und daß die Filteroperation gemäß Merkmal e zwischen diesen beiden Schritten eingebettet ist.

Eine bevorzugte Ausgestaltung der Erfindung ist in Anspruch 2 angegeben. Alle Strahlen, die die in Anspruch 2 definierte Fläche durchsetzen, liegen in zur Rotationsachse parallelen Ebenen, und diese Ebenen definieren miteinander einen Winkelbereich von exakt 180°. Die Schwächung in dieser Fläche kann daher ohne Verwendung redundanter Meßdaten rekonstruiert werden. Bei allen anderen Flächen müßten Strahlen aus einem größeren Winkelbereich herangezogen werden

Es sei erwähnt, daß aus der Veröffentlichung der Erfinder "Helical Cone Beam Tomography" in der Zeitschrift "International Journal of Imaging Systems and Technology", Vol 11, 2000, (p 91-100) ein Verfahren für die gefilterte Rückprojektion bekannt ist, bei dem die (der Rückprojektion vorangehende) eindimensionale Filterung die Meßwerte von Strahlen erfaßt, die eine die Rotationsachse enthaltende Ebene auf geneigten. Linien durchsetzen. Diese Linien nähern die Projektion einer als "Pi-surface" bezeichneten Fläche (die mit der im Anspruch 2 definierten Fläche identisch ist) auf die Ebene an. Allerdings ist die Projektion nur für einige Projektionsrichtungen linienförmig. Deshalb wird keine optimale Bildqualität eneicht, wenn man die Schwächung jeweils zweidimensional für eine Folge dieser Flächen rekonstruiert. Darüberhinaus erfolgen dort die Filterung und die Rückprojektion in der üblichen Reihenfolge, während bei Erfindung diese Schritte ineinander verschachtelt sind.

Die in Anspruch 3 angegebene Weiterbildung ist insbesondere bei relativ großen Abmessungen der Detektoreinheit in Richtung der Rotationsachse von Vorteil. Bei geringeren Abmessungen kann sie entfallen, weil die Cosinusfunktion dann für sämtliche Strahlen in guter Näherung den Wert 1 hat.

Wenn gemäß Anspruch 4 die Schwächungswerte an den Gitterpunkten eines regelmäßigen kartesischen Gitters ermittelt werden, können nachträglich auf einfache Weise CT-Bilder von beliebigen Flächen im Untersuchungsbereich erstellt werden.

Offensichtlich sind die Daten um so spezifischer für nur eine PI-Fläche, je größer der Winkelbereich ist, von dem Meßwerte für die Berechnung von Verbindungswerten herangezogen werden. Auf der anderen Seite ist die stückweise lineare Approximation der Trajektorien um so ungenauer, je größer der Winkelbereich ist, aus dem Meßwerte zur Berechnung von Verbindungswerten herangezogen werden. Ein günstiger Kompromiß zwischen diesen einander widersprechenden Forderungen ergibt sich bei der Ausgestaltung nach Anspruch 5.

Vorteilhafterweise werden gemäß Anspruch 6 die Strahlen einem sogenannten Parallel-Rebinning unterzogen.

Anspruch 7 beschreibt einen zur Durchführung des erfindungsgemäßen Verfahrens geeigneten Computertomographen und Anspruch 8 ein Computerprogramm zur Durchführung des erfindungsgemäßen Rekonstruktionsverfahrens.

Die Erfindung wird nachstehend anhand der Zeichnungen näher erläutert. Es zeigen:

- Figur 1 einen Computertomographen, mit dem das erfindungsgemäße Verfahren durchführbar ist,

- Figur 2 ein Ablauflaufdiagramm zur Erläuterung des erfindungsgemäßen Verfahrens,

- Figur 3 einen die Lage und die Orientierung der Strahlen beschreibenden zweidimensionalen Parameterraum,

- Figur 4 die Lage einer Verbindungslinie in einem solchen Parameterraum,

- Figur 5 mehrere solcher Verbindungslinien, die ein Teilstück der Trajektorie approximieren, die diejenigen Strahlen verbindet, die durch ein Pixel verlaufen,

- Figur 6 die der Erfindung zugrundeliegenden geometrischen Verhältnisse,

- Figur 7 die Projektion verschiedener Pixel auf eine die Rotationsachse enthaltenen Ebene,

- Figur 8 einen dreidimensionalen Paramterraum mit einem Netz von Verbindungslinien,

- Figur 9 die Verbindungslinien, die einer gemeinsamen Filteroperation unterzogen werden und

- Figur 10 die Lage der rekonstruierbaren Flächen relativ zu der von der Strahlenquelle beschriebenen helixförmigen Bahn.

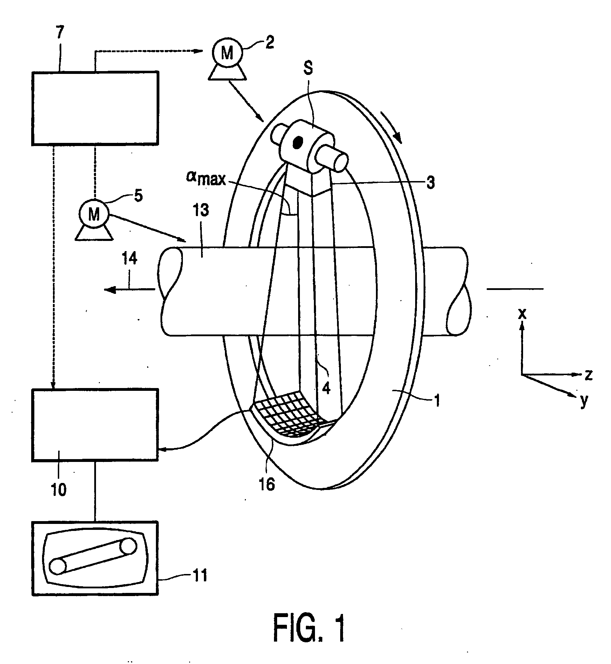

Der in Fig. 1 dargestellte Computertomograph umfaßt eine Abtasteinheit in Form einer Gantry 1, die um eine Rotationsachse 14 rotieren kann. Dazu wird die Gantry von einem Motor 2 mit einer vorzugsweise konstanten, aber einstellbaren Winkelgeschwindigkeit angetrieben. An der Gantry 1 ist eine Strahlenquelle S befestigt, beispielsweise ein Röntgenstrahler. Dieser ist mit einer Kollimatoranordnung 3 versehen, die aus der von der Strahlenquelle S erzeugten Strahlung ein kegelförmiges Strahlenbündel 4 ausblendet, dh ein Strahlenbündel, das sowohl in einer zur Rotationsachse senkrechten Ebene als auch in Richtung der Rotationsachse eine wn Null verschiedene endliche Ausdehnung hat.

Das Strahlenbündel 4 durchdringt einen Untersuchungsbereich 13, in dem sich ein Patient auf einem Patientenlagerungstisch (beides nicht näher dargestellt) befinden kann. Der Untersuchungsbereich 13 hat die Form eines Zylinders. Nach dem Durchsetzen dieses Zylinders trifft das Röntgenstrahlenbündel 4 auf eine an der Gantry 1 befestigte zweidimensionale Detektoreinheit 16, die eine Vielzahl von matrixförmig angeordneten Detektorelementen umfaßt. Jedes Detektorelement kann in jeder Strahlenquellenposition einen Meßwert für einen Strahl aus dem Strahlenbündel 4 liefern Die Detektorelemente sind in Zeilen und Spalten angeordnet. Die Detektorspalten verlaufen parallel zur Rotationsachse. Die Detektorzeilen können sich in zur Rotationsachse senkrechten Ebenen befinden, beispielsweise auf einem Kreisbogen um die Strahlenquelle S. Die Detektorzeilen enthalten in der Regel wesentlich mehr Detektorelemente (z.B. 1.000) als die Detektorspalten (zB. 16).

Der mit $\alpha_{max}$ bezeichnete Öffnungswinkel des Strahlenbündels 4 (als Öffnungswinkel ist der Winkel definiert, den ein Strahl, der in einer zur Rotationsachse 14 senkrechten Ebene am Rande des Strahlenbündels 4 liegt, mit einer durch die Strahlenquelle S und die Rotationsachse 14 definierten Ebene einschließt) bestimmt dabei den Durchmesser des Zylinders, innerhalb dessen sich das zu untersuchende Objekt bei der Akquisition der Meßwerte befinden muß. Das Untersuchungsobjekt bzw. der Patientenlagerungstisch kann mittels eines Motors 5 auch parallel zur Rotationsachse 14 verschoben werden. Die Geschwindigkeit dieses Vorschubs ist vorzugsweise konstant und einstellbar. Wenn die Motoren 5 und 2 gleichzeitig laufen, ergibt sich eine helixförmige Abtastbewegung der Strahlenquelle S und der Detektoreinheit 16.

Die von der Detektoreinheit 16 auf der rotierenden Gantry 1 akquirierten Meßdaten werden einem Bildverarbeitungsrechner 10 zugeführt, der sich in der Regel an einem festen Punkt im Raum befindet und mit der Detektoreinheit über einen kontaktlos arbeitenden, nicht näher dargestellten Datenschleifring verbunden ist. Der Bildverarbeitungsrechner 10 kann verschiedene Bildverarbeitungsoperationen durchführen. Unter anderem kann er aus den akquirierten Meßwerten die Schwächung der Röntgenstrahlung in dem Untersuchungsbereich 13 rekonstruieren, so daß sich ein 3D-Datensatz ergibt.

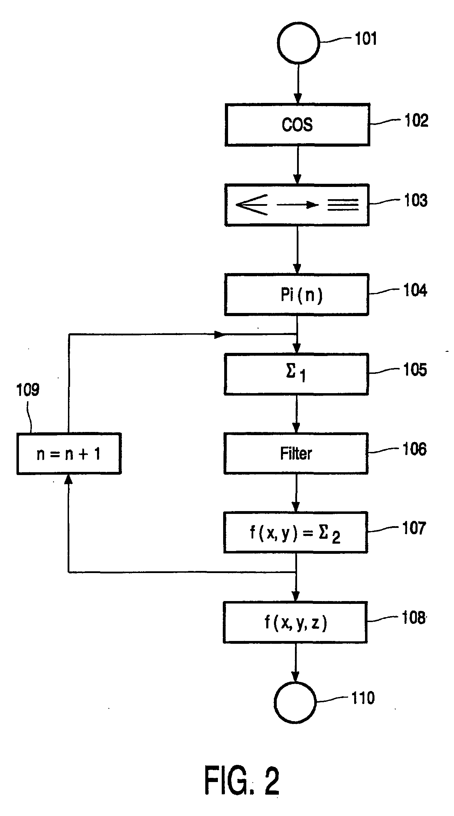

Im folgenden wird die Erfindung unter Bezugnahme auf das in Figur 2 dargestellte Ablaufdiagramm erläutert. Nach der Initialisierung (Block 101) sind die Motoren 2 und 5 gleichzeitig eingeschaltet, so daß sich die Strahlenquelle S auf einer helixförmigen Bahn relativ zum Untersuchungsobjekt bewegt. Dabei werden im Schritt 102 Meßwerte von der Detektoreinheit 16 akquiriert, die - gegebenenfalls nach einer Glättung und einer Logarithmierung - dem Linienintegral der Schwächung entlang des Strahles entsprechen, entlang dessen sie gemessen wurden. In einem dreidimensionalen $(\beta, \gamma, s)$ Parameterraum sind diese Meßwerte $p(\beta,\gamma,s)$ bzw. die zugehörigen Strahlen gekennzeichnet durch die Richtung $\beta$ eines Lotes von der jeweiligen Strahlenquellenposition auf die Rotationsachse 14 (wobei $\beta$ nach mehr als einem Umlauf größer werden kann als $2\pi$), durch den Winkel $\gamma$ ,den der zu dem jeweiligen Meßwert gehörende Strahl in einer zur Rotationsachse senkrechten Ebene mit dem erwähnten Lot einschließt, und durch die Höhenkoordinate s des Strahles (in einer zu dem Lot senkrechten und die Rotationsachse enthaltenden Ebene). Jeder Strahl ist durch einen Punkt in diesem dreidimensionalen Parameterraum gekennzeichnet.

Erforderlichenfalls können in diesem Ablaufschritt sämtliche Meßwerte mit dem Cosinus des Winkels gewichtet werden, den der zugehörige Strahl mit einer zur Rotationsachse senkrechten Ebene einschließt. Wenn der Cosinus aber für alle Strahlen praktisch den Wert 1 hat, kann der Schritt auch entfallen; er wird notwendig, wenn die Abmessungen der Detektoreinheit 16 gegenüber deren Abstand von der Strahlenquelle nicht mehr vernachlässigbar wird.

Im nächsten Schritt 103 kann ein sogenanntes Parallel-Rebinning erfolgen. Gemäß der Beziehung

$$p(\beta,\gamma,s) = p(\theta\text{-arcsin}\ \frac{t}{R},\ \text{arcsin}\ \frac{t}{R},\ s) \rightarrow p^P(\theta,t,s) \tag{1}$$

Der so entstandene neue Satz von Meßwerten $p^P(\theta,t,s)$ ist dabei durch die (Projektions-) Richtung $\theta$ des zu dem jeweiligen Meßwert gehörenden Strahl in einer zur Rotationsachse 14 senkrechten Ebene, durch den Abstand t dieses Strahls von der Rotationsachse 14 und durch die bereits erläuterte Koordinate s gekennzeichnet. Obwohl diese durch Umsortierung und Uminterpolation ermittelten neuen Meßwerte den ursprünglichen von Meßwerten $p(\beta,\gamma,s)$ äquivalent sind, werden sie im folgenden zur besseren Unterscheidung auch als Projektionswerte bezeichnet und mit dem hochgestellten Index P gekennzeichnet. Die zu diesen Meßwerten gehörenden Strahlen definieren in dem $(\theta, t, s)$ - Parameterraum ein regelmäßiges Gitter.

Im folgenden sollen zunächst anhand eines vereinfachten Beispiels das aus dem eingangs genannten Dokument bekannte Rekonstruktionsverfahren und das erfindungsgemäße Rekonstrukionsverfahren erläutert werden. Der Vereinfachung liegt die Annahme zugrunde, daß die Detektoreinheit lediglich eine einzige Zeile von Detektoren enthält, so daß s = 0 ist. Bei dem bekannten Verfahren erfolgt nach dem Rebinning in Filterschritt, bei dem alle Meßwerte mit dem gleichen Projektionswinkel $\theta$ und unterschiedlichem t einer gemeinsamen Filteroperation unterzogen werden.

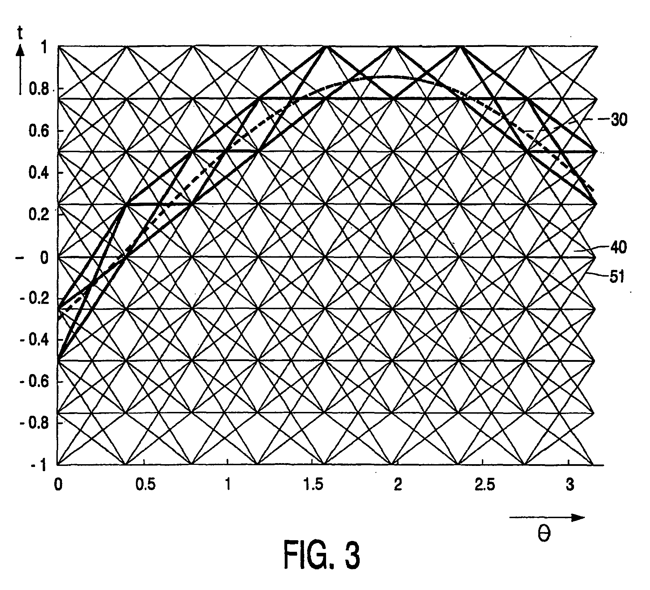

Erst danach erfolgt eine Rückprojektion der gefilterten Daten in den Ortsraum, wobei zur Berechnung des Schwächungswertes für einen Punkt im Untersuchungsbereich Beiträge von sämtlichen Strahlen summiert werden, die diesen Punkt passiert haben. Zu diesern Verfahrenschritt wird auf Figur 3 verwiesen, die den zweidimensionalen $(\theta,t)$ - Parameterraum mit dem Projektionswinkel $\theta$ als Abszisse und der Koordinate t darstellt. In diesen Parameterraum ist mit dicken, gestrichelten Linien eine Trajektorie 30 eingezeichnet, die- über einen Bereich des Projektionswinkels $\theta$ von 0 bis $\pi$ - die Strahlen miteinander verbindet, die durch den betreffenden Punkt verlaufen sind Diese Strahlen fallen in der Regel nicht mit einem der Gitterpunkte im $(\theta,t)$ -Parameterraum zusammen, an denen die Projektionswerte $p^P$ vorliegen. Deshalb müssen die der Rückprojektion zugrunde zu legenden Werte durch eine Interpolation ermittelt werden. Man bezeichnet die Darstellung gemäß Fig 3 wegen des Sinus-ähnlichen Verlaufs der Trajektorie 30 oft auch als Sinogramm.

Die Erfindung geht einen anderen Weg. Die bei der Rückprojektion entlang des gesamten Projektionswinkelbereiches von 0 bis $\pi$ vorgenommen Summierung wird in zwei Teilschritten vorgenommen, und der Filterschritt findet nicht vor der Rückprojektion, sondern zwischen den beiden Teilschritten der Rückprojektion statt. Dazu wird in dem Parameterraum ein Netz 40 gebildet, dessen Knoten in $\theta$- und t-Richtung einen wessentlich größeren Abstand haben als die Gitterpunkte des kubischen Gitters, an denen Projektionswerte definiert sind Benachbarte Spalten von Knoten sind dabei durch Verbindungslinien (engl.: links) 51 miteinander verbunden. Einige dieser Verbindungslinien 51 haben eine Steigung, die mindestens so groß ist wie die größte Steigung einer Trajektorie 30 in dem Sinogramm. Jede beliebige Trajektorie kann dann durch eine Satz von Verbindungslinien approximiert werden. In Figur 3 sind diejenigen Verbindungslinien, die die Trajektorie 30 approximieren, fett dargestellt.

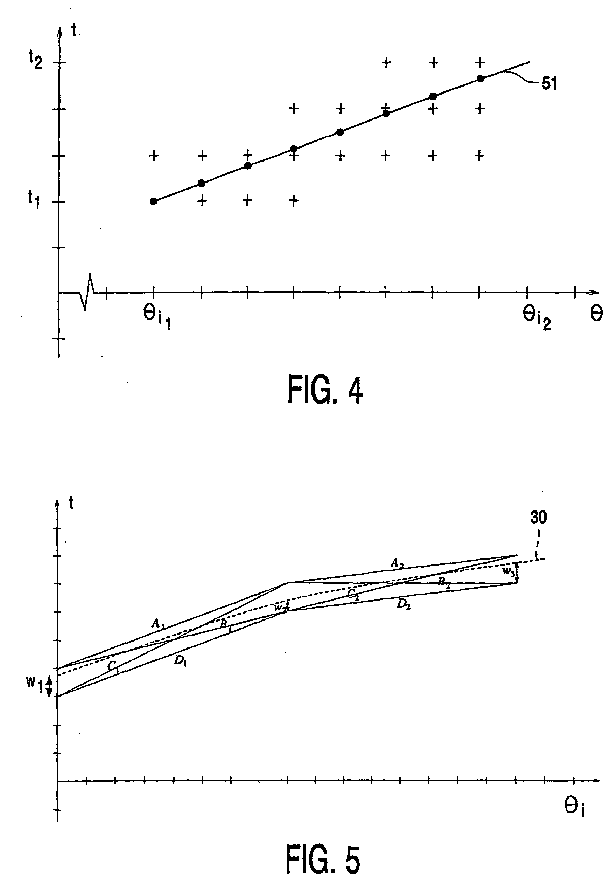

Die erste Teilsummation erfolgt dadurch, daß Verbindungswerte berechnet werden, indem die Projektionswerte entlang jeder der Verbindungslinien des Netzes berechnet und summiert werden. Dies wird anhand von Figur 4 erläutert, die einen Ausschnitt aus dem Sinogramm nach Figur 3 darstellt, sowie eine einzige Verbindungslinie 51, die die Punkte ($\theta_{i1}$, $t_1$ und $\theta_{i2}$, $t_2$) miteinander verbindet. Einige der Gitterpunkte, an denen die Werte $p^P$ definiert sind, sind in Fig. 4 durch Kreuze markiert. Wie Figur 4 zeigt, fallen die Gitterpunkte und die sich für jedes Projektionswinkel-Inkrement ergebenden Punkte auf der Verbindungslinie nicht miteinander zusammen. Auch die Endpunkte der Verbindungslinie 51 müssen nicht notwendigerweise mit einem Gitterpunkt zusammenfallen. Die Berechnung des Verbindungswertes $I(\theta_{i_1},t_1;\theta_{i_2},t_2)$ für die Verbindungslinie 51 zwischen den Punkten ($\theta_{i_1},t_1$) und ($\theta_{i_2},t_2$) erfolgt nach der Gleichung

$$I(\theta_{i_1},t_1;\theta_{i_2},t_2) = \sum_{i=i_1}^{i_2-1} p^P\left(\theta_i,t_1 + \frac{i-i_1}{i_2-i_1}(t_2-t_1)\right) \tag{2}$$

Durch den noch zu erläuternden anschließend vorgenommenen Filterschritt ergeben sich aus den Verbindungswerten Filterdaten. Bei dem zweiten Summationsschritt werden gemäß Fig. 5 die Filterdaten von denjenigen Verbindungslinien $A_1.....D_1$ und $A_2.....D_2$ summiert, die die Trajektorie 30 approximieren. Fig. 5 stellt nur einen Teil der Trajektorie und der Verbindungslinien dar. Der Schwächungswert f(x,y) für den Punkt (x,y) - im folgenden als Pixel bezeichnet -, dem die Trajektorie 30 zugeordnet ist, berechnet sich nach der Beziehung

$$f(x,y) = \sum_{j=1}^{k}\left(w_j\left(w_{j+1}A_j + (1-w_{j+1})B_j\right) + (1-w_j)\left(w_{j+1}C_j + (1-w_{j+1})D_j\right)\right) \tag{3}$$

Dabei sind $A_j,B_j.....D_j$ die Filterdaten für die Verbindungslinien, mit denen die Trajektorie 30 angenähert wird, und k ist die Zahl der Projektionsintervalle, in die das Sinogramm durch das Netz 40 unterteilt wird. Die Werte $w_j$ und $w_{j+1}$ sind vom Abstand der Verbindungslinien von der Trajektorie 30 abhängige Gewichtungsfaktoren, deren Bedeutung sich aus Figur 5 ergibt. Die Zahl k der Projektionsintervalle ändert sich im umgekehrten Verhältnis zur Länge der Projektionsintervalle. Große k-Werte entsprechen einem feinmaschigen Netz und einer guten Approximation der Trajektorien durch die Verbindungslinie; sie erfordern aber auch einen hohen Rechenaufwand. Ein günstiger Kompromiß zwischen Genauigkeit und Rechenaufwand ergibt sich nach der Beziehung $k \approx \sqrt{N_\theta}$. Dabei ist $N_\theta$ die Zahl der gleichmäßig über 180° verteilten Projektionswinkel, an denen die Projektionswerte in dem dreidimensionalen Parameterraum definiert sind.

Da man die einzelnen Verbindungslinien auch für die Approximation der Trajektorien von anderen Trajektorien benutzen kann, werden die zugehörigen einmal ermittelten Filterdaten mehrfach benutzt, was den Rechenaufwand einschränkt.

Im folgenden wird dieses Verfahren für einen dreidimensionalen Datensatz näher erläutert. Im dreidimensionalen Fall gibt es ja keine durch die Messung in irgend einer Weise hervorgehobene Fläche, wie im zweidimensionalen Fall. Trotzdem wird im Schritt 104 eine Fläche vorgegeben, für deren Pixel die Schwächung der Strahlung rekonstruiert wird.

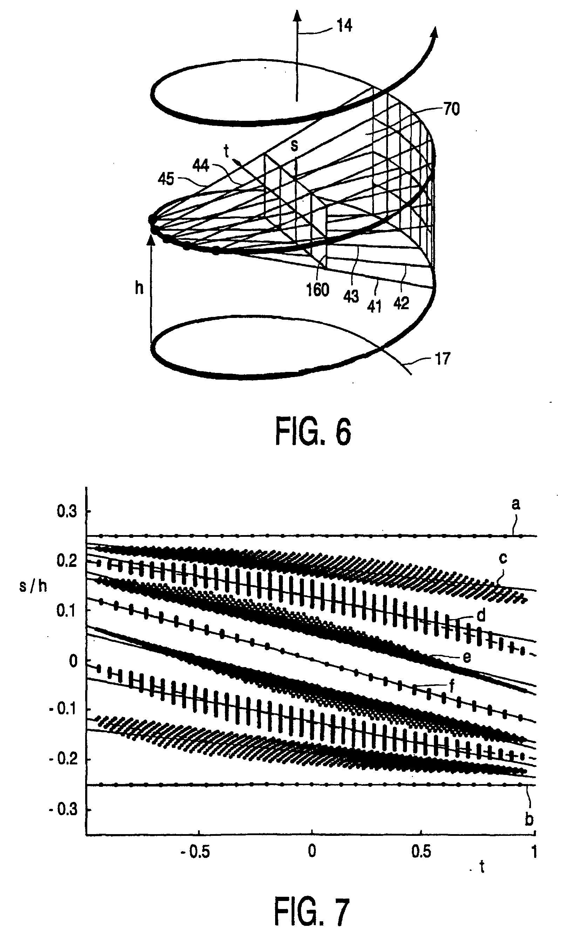

Hierzu wird auf Figur 6 verwiesen, die die helixförmige Bahn 17 der Strahlenquelle relativ zum Untersuchungsbereich darstellt, wobei angenommen ist, daß die Strahlenquelle sich von unten nach oben bewegt. Weiterhin sind für einen bestimmten Projektionswinkel einige Strahlenfächer 41, 42 ... 45 dargestellt, die den nicht näher dargestellten Untersuchungsbereich in zueinander und zur Rotationsachse 14 parallelen Ebenen durchsetzen. Dabei ist angenommen, daß die oberen und unteren Randstrahlen die gegenüberligenden Segmente der Helix 17 durchstoßen. Wenn die Detektoreinheit so gestaltet ist, daß Meßwerte nur für Strahlen in dem Berech zwischen benachbarten Helixwindungen erfaßt werden (oder wenn die Meßwerte nur von diesen Strahlen ausgewertet werden), ist - wie aus dem eingangs genannten Dokument bekannt - sichergestellt, daß jeder Punkt im Untersuchungsbereich aus einem Projektionswinkelbereich von exakt 180° bestrahlt wird.

Weiter ist in der Zeichnung eine die Rotationsachse 14 enthaltende, zur Projektiorsrichtung senkrechte rechteckige Fläche 160 dargestellt. Die obere und untere Seite dieses Rechtecks fällt mit den oberen und unteren Randstrahlen der parallelen Strahlenfächer zusammen. Die linke und rechte Seite sind durch den äußeren Rand des Untersuchungsbereichs 13 (vgl. Figur 1) definiert.

Da somit alle Strahlen für eine bestimmte Positionsrichtung durch das Rechteck 160 verlaufen, wird dieses im folgenden auch als Detektorfenster bezeichnet. Bei anderen Projektionsrichtungen ändert dieses Detektorfenster seine Lage und Orientierung im Raum im gleichen Maße wie die Strahlenfächer, die es durchsetzen. Wenn man annimmt, daß sich die Strahlenquelle auf der Helix 17 nach oben bewegt, werden alle Punkte im Untersuchungsbereich zunächst auf den oberen Rand des Detektorfensters projiziert, und nach einer Änderung des Projektionswinkels von 180° passieren sie den unteren Rand Die Strahlen, die in der Darstellung der Figur 6 den oberen Rand des Detektorfensters 6 passieren - wie die Strahlen 44 und 45 -, verlaufen nach einer Zunahme der Projektionsrichtung um 180° durch den unteren Rand des (dann entsprechend seiner Höhe nach oben verschobenen) Detektorfensters 160.

Im Schritt 104 wird diese Fläche 70, die durch gegenüberliegende Segmente der Helix 17 miteinander verbindende und in parallelen Ebenen verlaufende Strahlen gebildet wird, vorgegeben. Diese - nicht ebene - Fläche wird in der Literatur auch als PI-Fläche bezeichnet (engl.: pi-surface), weil im $(\theta t,s)$ - Parameterraum *alle* Projektionswerte $p^P$, die Strahlen zugeordnet sind, die diese Fläche durchsetzen bzw. in ihr liegen, sich innerhalb eines Projektionswinkel-Bereichs von exakt 180° befinden.

Nur Strahlen, die in dieser Fläche liegen bzw. die diese Fläche durchsetzen, werden einer gemeinsamen Filteroperation unterzogen, und nur für diese Fläche erfolgt zunächst eine zweidimensionale Rekonstruktion der Schwächung - Man könnte auch eine andere Fläche vorgeben, zB. eine ebene Fläche. Dann müßten aber Projektionswerte $p^P$ aus einem Projektionswinkel-Bereich von mehr als 180° herangezogen werden, was die Filterung und die Rekonstruktion erschweren würde.

Fig 7 stellt die Projektion der Pixel in einer PI-Fläche für jeweils um 22,5° gegeneinander versetzte Projektionsrichtungen auf das Detektorfenster 160 dar. Wie bereits erwähnt, fallen die Projektionen der Pixel in der PI-Fläche beim Eintritt in das Strahlenbündel mit dem oberen Rand a und beim Austritt mit dem unteren Rand b des Detektorfensters 160 zusammen. Der obere Rand a des Detektorfensters verläuft dabei entsprechend der Beziehung s/h = 0,25 horizontal ebenso wie der untere Rand b nach der Beziehung s/h = -0,25, wobei h der Abstand zwei benachbarter Helixwindungen ist. Für die dazwischen liegenden Projektionsrichtungen werden die Pixel der PI-Fläche 70 nicht mehr auf eine Gerade projiziert, sondern auf schmale Streifen - c, d, e, f usw. -, die gegenüber den horizontalen Geraden a und b mehr oder weniger geneigt sind. Die Streifen von benachbarten Projektionsrichtungen können einander überlappen.

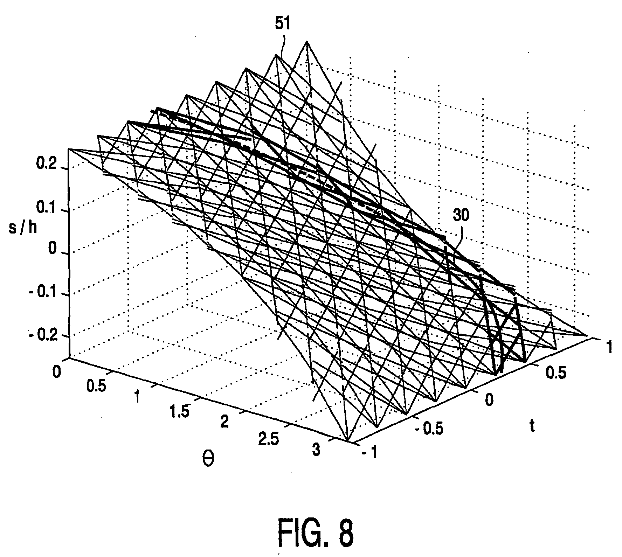

Das Netz 40, das in dem $(\theta, t, s)$ -Parameterraum diejenigen Strahlen umfaßt, die die PI-Fläche durchsetzen, befindet sich also nicht mehr in einer Ebene (wie im zweidimensionalen Fall), sondern auf einer gekrümmten Fläche. Die Projektion dieses Teils des Netzes auf eine $(\theta,t)$ -Ebene entspricht zwar der Darstellung nach Figur 3; doch haben die Knoten dieses Netzes unterschiedliche s-Koordinaten. Eine Verbindungslinie 51, die zwei Strahlen (bzw. Knoten des Netzes 40) mit den Koordinaten $\theta_1$, $t_1$ und $\theta_2$, $t_2$ miteinander verbindet, hat Koordinaten $s_1$ und $s_2$. Diese beiden Strahlen schneiden sich in einem Pixel auf der PI-Fläche mit den Koordinaten x,y. Die Projektion dieses Pixels (x,y) aus den Projektionsrichtungen $\theta_1$ and $\theta_2$ auf das Detektorfenster 160 ergibt dann die zu der Verbindungslinie 51 gehörenden Koordinaten $s_1$ und $s_2$ .In Figur 8 ist der erwähnte Teil des Netzes im $(\theta,t,s)$-Parameterraum perspektivisch dargestellt.

Im Schritt 105 werden die Verbindungswerte für dies Verbindungslinien dieses Netzes berechnet. Die Gleichung 2 geht dann über in

$$I(\theta_{i_1},t_1,\theta_{i_2},t_2,) = \sum_{i=i_1}^{i_2-1} p^P\left(\theta_i, t_1 + \frac{i-i_1}{i_2-i_1}(t_2 - t_1), s_1 + \frac{i-i_1}{i_2-i_1}(s_2 - s_i)\right) \qquad (4)$$

Im Schritt 106 erfolgt dann eine Filterung der Verbindungswerte von Verbindungslinien, die zu dem in Figur 8 dargestellten Teil des Netzes gehören, nach der Beziehung

$$\tilde{I}(\theta_{i_1},t_1;\theta_{i_2},t_2) = I(\theta_{i_1},t_1,\theta_{i_2},t_2,) \otimes g(t) \qquad (5)$$

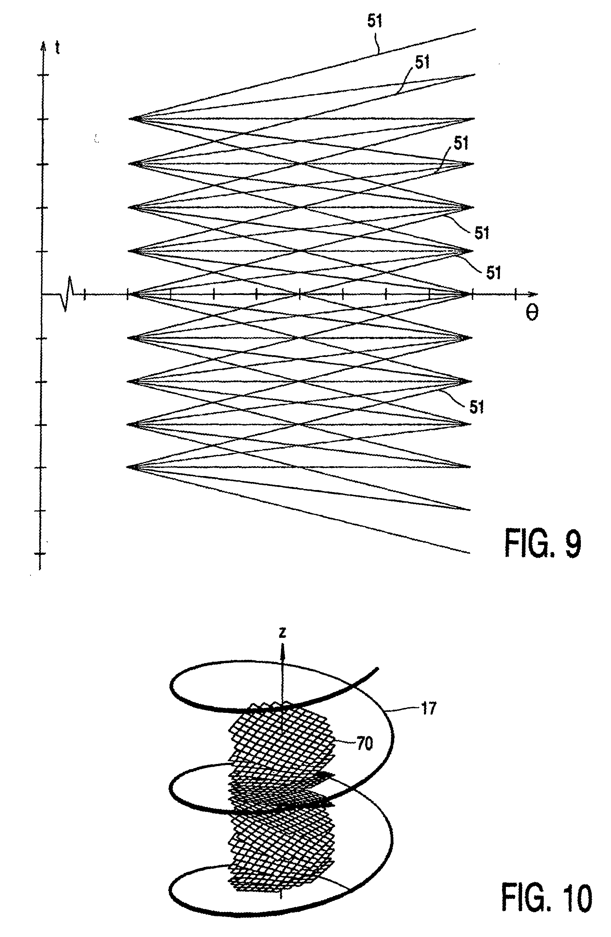

wobei der Operator $\otimes$ eine Faltungsoperation symbolisiert und g(t) eine Filterfunktion ist die durch die Faltung die gewünschte rampenförmige Filterung bewirkt. Gleichung 5 stellt eine eindimensionale Faltung dar, bei der alle Verbindungslinien, die von dem gleichen Projektionswinkel $(\theta_{i1})$ ausgehen und die die gleiche Steigung $\Delta t = t_2 - t_1$ haben, einer (gemeinsamen) Filterung unterzogen werden. Dies ist in Figur 9 dargestellt, die die von einem bestimmten Projektionswinkel θ ausgehenden Verbindungslinien zeigt, wobei Linien gleicher Steigung auf dieselbe Weise gestrichelt bzw. punktiert dargestellt sind. Eine Filteroperation beispielsweise umfaßt die Verbindungswerte der mit 51 geasichneten Verbindungslinien.

Im nachfolgenden Schritt 107 werden die Filterdaten von Verbindungslinien summiert, die die Trajektorien 30 (vgl. Figur 8) der einzelnen Pixel (x, y) der PI-Fläche 70 (Figur 6) approximieren. Alle Trajektorien von Pixeln in dieser PI-Fläche starten auf der Geraden s/h = 0,25 und θ = θ$_i$ (bei Figur 8 ist θ$_i$ = 0). Alle Trajektorien enden auch in einer gemeinsamen Linie, die durch die Koordinaten s/h = -0,25 und θ = θ$_i$ + π bestimmt ist. Die Becechnung erfolgt nach Gleichung 3, wobei die Werte A$_j$... D$_j$ durch die im Schritt 106 berechneten Filterdaten $\tilde{i}(\theta_{i_1}, t_1; \theta_{i_2}, t_2)$ gebildet werden, die die Trajektorie 30 approximieren.

Nach dem Schritt 107 sind die Schwächungswerte f(x,y) auf der im Schritt 104 vorgegebenen PI-Fläche 70 gegeben. Im darm folgenden Schritt 109 wird eine andere PI-Fläche vorgegeben, die sich von der vorigen PI-Fläche beispielsweise um ein Inkrement der Projektionsrichtung unterscheidet und in Richtung der Rotationsachse 14 versetzt ist. Die Schritte 105 bis 107 werden für diese neue PI-Fläche erneut ducchlaufen und danach für weitere PI-Flächen wiederholt. Zum Schluß sind die Schwächungswerte für eine Anzahl von in z-Richtung gegeneinander versetzten und um Inkremente des Projektionswinkels gegeneinander gedrehten PI-Flächen ermittelt, die in Figur 10 dargestellt sind.

Um die räumliche Verteilung der Schwächung f(x,y,z) in dem durch die beiden äußersten PI-Flächen definierten dreidimensionalen Bereich an den Punkten eines regelmäßigen kartesischen Gitters, vorzugsweise eines kubischen Gitters, darstellen zu können, wird im Schritt 108 eine Interpolation durchgeführt. Da schon die Schwächungswerte für die einzelnen PI-Flächen für jeweils dieselben x- und y-Koordinaten berechnet werden, ist lediglich eine Interpolation in z-Richtung erforderlich. Danach wird das Verfahren beendet (Schritt 110).

Wenn das im Schritt 103 gemäß Gleichung 1 erfolgende Rebinning verlagert wird auf die in Schritt 104 erfolgende Berechnung der Verbindungswerte nach Gleichung 4, dann können. die Verbindungswerte direkt aus den Meßwerten $p(\beta,\gamma,s)$ berechnet werden, ohne daß eine Rebinning erforderlich wäre. Das Rebinning im Schritt 103 könnte dann also entfallen.

Weiterhin sind bei einer Detektoreinheit mit im zur Rotationsachse (bzw zur z-Achse) senkrecht verlaufenden Ebenen liegenden Detektorzeilen die Werte s für eine Detektorzeile nicht konstant, wenn die Detektorzeilen beispielsweise auf einem Kreisbogen um die Strahlenquelle angeordnet sind. Auch in diesem Fall ist ein Rebinning, das zu konstanten Werten s im dreidimensionalen Parameterraum führt, nicht erforderlich. Statt dessen kann eine Interpolation für jeden Summanden in der Gleichung 4 durchgeführt werden. Um Aliasing-Effekte zu vermeiden, sollten die Inkremente des Projektionswinkels, die der Gleichung 4 zugrunde liegen, verringert werden.

Die Berechnung der Verbindungswerte im Schritt 105 muß nicht auf je eine Fläche beschränkt sein, wie im Verfahren nach Fig. 2 angenommen. Es müßten dann aber sämtliche Verbindungswerte berechnet werden, die notwendig sind, bevor die Vorgabe einer (PI-) Fläche erfolgt und die dazu gehörenden Verbindungswerte gefiltert werden.

**Patentansprüche**

1. Computertomographie-Verfahren mit den Schritten

    a) Erzeugen eines kegelförmigen Strahlenbündels (4), das eine Vielzahl von Strahlen enthält, die von einer Strahlenquelle (S) ausgehen und einen Untersuchungsbereich (13) bzw. ein darin befindliches Objekt durchsetzen,
    b) Erzeugung einer Relativbewegung zwischen der Strahlenquelle (S) einerseits und dem Untersuchungsbereich (13) bzw. dem Objekt and ererseits, die eine Rotation um eine Rotationsachse (14) und eine Verschiebung parallel zur Rotationsachse umfaßt und die Form einer Helix (17) hat,
    c) Akquisition von Meßwerten, die von der Schwächung der Strahlen in dem Untersuchungsbereich abhängen, mit einer Detektoreinheit (16) während der Relativbewegung,
    **gekennzeichnet durch** die weiteren Schritte
    d) Berechnung von Verbindungswerten ($l(\theta_{i_1}, t_1; \theta_{i_2}, t_2,)$) **durch** Summation der Meßwerte entlang von Verbindungslinien (51) eines Netzes (40) in einem dreidimensionalen, die Lage und die Orientierung der Strahlen beschreibenden Parameterraum ($\theta,t,s$),
    e) Filtern der Verbindungswerte ($\tilde{i}(\theta_{i_1},t_1; \theta_{i_2},t_2,)$) zur Erzeugung von Filterdaten ($\tilde{i}(\theta_{i_1}, t_1; \theta_{i_2},t_2)$) für Verbindungslinien (51), die Strahlen zugeordnet sind, welche eine bestimmte Fläche (70) des Untersuchungsbereiches durchsetzen,
    f) Berechnung der Schwächung (f(x,y,z)) der Strahlung in Pixeln auf der Fläche (70) **durch** Summation der Filterdaten ($\tilde{i}(\theta_{i_1}, t_1; \theta_{i_2}, t_2)$) von Verbindungslinien, die die Trajektorie (30) approximieren, die in dem Parameterraum ($\theta,t,s$) **durch** die Strahlen definiert wird, die das jeweilige Pixel durchsetzen,
    g) Wiederholung zumindest der Schritte e) und f) für andere Flächen (70), die in Richtung der Rotationsachse gegeneinander versetzt sind.

2. Computertomographie-Verfahren nach Anspruch 1,
**dadurch gekennzeichnet,**
**dass** die Flächen (70) durch je eine Schar von Strahlen (41....45) definiert sind, die in zur Rotationsachse (14) parallelen Ebenen liegen und gegenüberliegende Abschnitte der Helix (17) miteinander verbinden.

3. Computertomographie-Verfahren nach Anspruch 1,
**gekennzeichnet durch** eine Wichtung der Meßwerte mit dem Cosinus des Winkels, den der jeweils zu dem Meßwert gehörende Strahl mit einer zur Rotationsachse senkrechten Ebene einschließt.

4. Computertomographie-Verfahren nach Anspruch 1,
**gekennzeichnet durch** die Berechnung der Schwächungswerte (f(,x,y,z)) an den Gitterpunkten eines regelmäßigen, dreidimensionalen Gitter, vorzugsweise eines kubischen Gitters, aus den Schwächungswerten (f(,x,y)) der Pixel (x,y) auf den Flächen (70).

5. Computertomographie-Verfahren nach Anspruch 1,
wobei zur Berechnung jedes Verbindungswertes Meßwerte aus einem Winkelbereich von $180°/N_1$ herangezogen werden, wobei $N_1$ zumindest näherungsweise den Wert hat $\sqrt{N_\theta}$ und $N_\theta$ die Zahl der über einen Winkelbereich von 180° verteilten Positionen der Strahlenquelle ist, in denen Meßwerte erfaßt werden.

6. Computertomographie-Verfahren nach Anspruch 1 mit einem solchen Rebinning der akquirierten Meßwerte ($p(\beta, \gamma,s)$), daß sich ein neuer Satz von Strahlen zugeordneten Meßwerten ($p^P(\theta,t,s)$) ergibt, die sich auf einem kartesischen Gitter in einem durch die Richtung ($\theta$) der Strahlen in einer zur Rotationsachse senkrechten Ebene und durch deren Lage ($t, s$) in einer die Rotationsachse (14) enthaltenden Ebene (160) definierten Parameterraum ($\theta, t,s$) befinden.

7. Computertomograph zur Durchführung des Verfahrens nach Anspruch 1

- mit einer Strahlenquelle (S) zur Erzeugung eines kegelförmigen Strahlenbündels (4), das einen Untersuchungsbereich (13) bzw. ein darin befindliches objekt durchsetzt,
- mit einer Antriebs-Anordnung (2,5) zur Erzeugung einer Relativbewegung zwischen der Strahlenquelle (S) und dem Untersuchungsbereich in Form einer Helix (17),
- mit einer Detektoreinheit (16) zur Akquisition von Meßwerten, die von der Schwächung der Strahlen in dem Untersuchungsbereich abhängen,
- und mit einer Rekonstruktionseinheit (10) zur Ermittlung der räumlichen Verteilung der Schwächungswerte (f, x,y,z) im Untersuchungsbereich, **dadurch gekennzeichnet, daß** die Rekonstruktionseinheit die Meßwerte entsprechend folgendem Ablauf verarbeitet:

a) Berechnung von Verbindungswerten ($l(\theta_{i_1}, t_1; \theta_{i_2}, t_2,)$) durch Summation der Meßwerte entlang von Verbindungslinien (51) eines Netzes (40) in einem dreidimensionalen, die Lage und die Orientierung der Strahlen beschreibenden Parameterraum ($\theta,t,s$),
b) Filtern der Verbindungswerte ($l(\theta_{i_1}, t_1; \theta_{i_2}, t_2,)$) zur Erzeugung von Filtendaten ($\tilde{l}(\theta_{i_1}, t_1; \theta_{i_2}, t_2)$) für Verbindungslinien (51) die Strahlen zugeordnet sind, welche eine bestimmte Fläche (70) des Untersuchungsbereiches durchsetzen,
c) Berechnung der Schwächung (f(x,y,z)) der Strahlung in Pixeln auf der Fläche (70) durch Summation der Filterdaten ($\tilde{l}(\theta_{i_1}, t_1; \theta_{i_2}, t_2)$) von Verbindungslinien, die die Trajektorie (30) approximieren, die in dem Parameterraum ($\theta,t,s$) durch die Strahlen definiert wird, die das jeweilige Pixel durchsetzen,
d) Wiederholung zumindest der Schritte e) und f) für andere Flächen (70), die in Richtung der Rotationsachse gegeneinander versetzt sind.

8. Computerprogramm zur Verarbeitung der Meßwerte eines Computertomographen

- mit einer Strahlenquelle (S) zur Erzeugung eines kegelförmigen Strahlenbündels (4), das einen Untersuchungsbereich (13) bzw. ein darin befindliches Objekt durchsetzt,
- mit einer Antriebs-Anordnung (2,5) zur Erzeugung einer Relativbewegung zwischen der Strahlenquelle (S) und dem Untersuchungsbereich in Form einer Helix (17),
- mit einer Detektoreinheit (16) zur Akquisition von Meßwerten, die von der Schwächung der Strahlen in dem Untersuchungsbereich abhängen,

- und mit einer Rekonstruktionseinheit (10) zur Ermittlung der räumlichen Verteilung der Schwächungswerte (f, x,y,z) im Untersuchungsbereich

**gekennzeichnet durch** die Schritte

a) Berechnung von Verbindungswerten ($l(\theta_{i_1}, t_1, \theta_{i_2}, t_2,)$) **durch** Summation der Meßwerte entlang von Verbindungslinien (51) eines Netzes (40) in einem dreidimensionalen, die Lage und die Orientierung der Strahlen beschreibenden Parameterraum ($\theta,t,s$),

b) Filtern der Verbindungswerte ($l(\theta_{i_1}, \theta_{i_2}, t_2,)$) zur Erzeugung von Filterdaten ($\tilde{l}(\theta_{i_1}, t_1; \theta_{i_2}, t_2)$) für Verbindungslinien (51), die Strahlen zugeordnet sind, welche eine bestimmte Fläche (70) des Untersuchungsbereiches durchsetzen,

c) Berechnung der Schwächung (f(x,y,z)) der Strahlung in Pixeln auf der Fläche (70) **durch** Summation der Filterdaten ($\tilde{l}(\theta_{i_1}, t_1; \theta_{i_2}, t_2)$) von Verbindungslinien, die die Trajektorie (30) approximieren, die in dem Parameterraum ($\theta,t,s$) **durch** die Strahlen definiert wird, die das jeweilige Pixel durchsetzen,

d) Wiederholung zumindest der Schritte e) und f) für andere Flächen (70), die in Richtung der Rotationsachse gegeneinander versetzt sind.

## Claims

1. A computed tomography method which includes the steps of

   (a) generating a conical radiation beam (4) which contains a plurality of rays that emanate from a radiation source (S) and traverse an examination zone (13) or an object present therein,

   (b) generating a relative motion between the radiation source (S) on the one side and the examination zone (13) or the object on the other side, which relative motion includes a rotation about an axis of rotation (14) and a displacement parallel to the axis of rotation and is shaped as a helix (17),

   (c) acquiring measured values, using a detector unit (16), which are dependent on the attenuation of the rays in the examination zone during the relative motion,

   **characterized in that** it also comprises the steps of

   (d) calculating link values ($l(\theta_{i1},t_1;\theta_{i2},t_2)$) by summing the measured values along links (51) of a network (40) in a three-dimensional parameter space ($\theta,t,s$) describing the position and orientation of the rays,

   (e) filtering the link values ($l(\theta_{i1},t_1;\theta_{i2},t_2)$) in order to produce filter data ($\tilde{l}(\theta_{i_1}, t_1; \theta_{i_2}, t_2)$) for links (51) that are associated with rays that pass through a given surface (70) of the examination zone,

   (f) calculating the attenuation (f(x,y,z)) of the radiation in pixels on the surface (70) by summing the filter data ($\tilde{l}(\theta_{i_1}, t_1; \theta_{i_2}, t_2)$) of links which approximate the trajectory (30) that is defined in the parameter space ($\theta,t,s$) by the rays that pass through the relevant pixel,

   (g) repeating at least the steps e) and f) for other surfaces (70) that are mutually offset in the direction of the axis of rotation.

2. A computed tomography method as claimed in claim 1, **characterized in that** each of the surfaces (70) is defined by a respective set of rays (41 ... 45) which are situated in planes extending parallel to the axis of rotation (14) and link oppositely situated segments of the helix (17).

3. A computed tomography method as claimed in claim 1, **characterized in that** the measured values are weighted with the cosine of the angle enclosed by the ray associated with the respective measured value relative to a plane extending perpendicularly to the axis of rotation.

4. A computed tomography method as claimed in claim 1, **characterized in that** the attenuation values (f(x,y,z)) are calculated at the grid points of a regular, three-dimensional grid, preferably a cubic grid, from the attenuation values (f(x,y)) of the pixels (x,y) on the surfaces (70).

5. A computed tomography method as claimed in claim 1, wherein measured values are taken from an angular range of $180°/N_1$ in order to calculate each link value, where $N_1$ has at least approximately the value $\sqrt{N_\theta}$ and $N_\theta$ is the number of positions of the radiation source which are distributed over an angular range of 180° and in which measured values are acquired.

**6.** A computed tomography method as claimed in claim 1, wherein the acquired measured values (p(β,γ,s)) are rebinned in such a manner that there is formed a new set of measured values ($p^P(\theta,t,s)$) which are associated with rays and are situated on a cartesian grid in a parameter space (θ,t,s) which is defined by the direction (θ) of the rays in a plane perpendicular to the axis of rotation and by their position (t,s) in a plane containing the axis of rotation (14).

**7.** A computed tomography apparatus for carrying out the method claimed in claim 1, which apparatus includes

- a radiation source (S) for generating a conical radiation beam (4) which traverses an examination zone (13) or an object present therein,
- a drive device (2, 5) for producing a relative motion in the form of a helix (17) between the radiation source (S) and the examination zone,
- a detector unit (16) for the acquisition of measured values which are dependent on the attenuation of the rays in the examination zone,
- and a reconstruction unit (10) for determining the spatial distribution of the attenuation values (f,x,y,z) in the examination zone,

**characterized in that** the reconstruction unit processes the measured values as follows:

a) calculating link values ($I(\theta_{i1},t_1;\theta_{i2},t_2)$) by summing the measured values along links (51) of a network (40) in a three-dimensional parameter space (θ,t,s) which describes the position and the orientation of the rays,
b) filtering the link values ($I(\theta_{i1},t_1;\theta_{i2},t_2)$) in order to generate filter data ($\tilde{I}(\theta_{i_1}, t_1; \theta_{i_2}, t_2)$) for links (51) which are associated with rays passing through a given surface (70) of the examination zone,
c) calculating the attenuation (f(x,y,z)) of the radiation in pixels on the surface (70) by summing the filter data ($\tilde{I}(\theta_{i_1}, t_1; \theta_{i_2}, t_2)$) of links which approximate the trajectory (30) which is defined in the parameter space (θ,t,s) by the rays passing through the relevant pixel,
d) repeating at least the steps e) and f) for other surfaces (70) which are mutually offset in the direction of the axis of rotation.

**8.** A computer program for processing the measured values of a computed tomography apparatus comprising:

- a radiation source (S) for generating a conical radiation beam (4) which traverses an examination zone (13) or an object present therein,
- a drive device (2, 5) for producing a relative motion in the form of a helix (17) between the radiation source (S) and the examination zone,
- a detector unit (16) for the acquisition of measured values which are dependent on the attenuation of the rays in the examination zone,
- and a reconstruction unit (10) for determining the spatial distribution of the attenuation values (f, x, y, z) in the examination zone,

**characterized in that** it comprises the steps of:

a) calculating link values ($I(\theta_{i1},t_1,\theta_{i2},t_2)$) by summing the measured values along links (51) of a network (40) in a three-dimensional parameter space (θ,t,s) which describes the position and the orientation of the rays,
b) filtering the link values ($I(\theta_{i1},t_1,\theta_{i2},t_2)$) in order to generate filter data ($\tilde{I}(\theta i_1, t_1; \theta_{i_2}, t_2)$) for links (51) which are associated with rays passing through a given surface (70) of the examination zone,
c) calculating the attenuation (f(x,y,z)) of the radiation in pixels on the surface (70) by summing the filter data ($\tilde{I}(\theta_{i_1}, t_1; \theta_{i_2}, t_2)$) of links which approximate the trajectory (30) which is defined in the parameter space (θ,t,s) by the rays passing through the relevant pixel,
d) repeating at least the steps e) and f) for other surfaces (70) which are mutually offset in the direction of the axis of rotation.

**Revendications**

**1.** Procédé de tomographie informatisée comportant les étapes suivantes:

a) production d'un faisceau conique de rayons (4) qui contient une multitude de rayons qui sortent d'une source de rayons (S) et traversent une zone d'examen (13) ou un objet qui s'y trouve,

b) production d'un mouvement relatif entre la source de rayons (S), d'une part, et la zone d'examen (13) ou l'objet, d'autre part, qui comprend une rotation autour d'un axe de rotation (14) et un coulissement parallèle à l'axe de rotation et a la forme d'une hélice (17),

c) acquisition de valeurs de mesure qui dépendent de l'affaiblissement des rayons dans la zone d'examen, avec une unité de détection (16) pendant le mouvement relatif,

**caractérisé par** les étapes supplémentaires suivantes:

d) calcul des valeurs de liaison ($l(\theta_{i1}, t_1; \theta_{i2}, t_2)$) par addition des valeurs de mesure le long de lignes de liaison (51) d'un réseau (40) dans un espace de paramètres ($\theta$, $t$, s) tridimensionnel décrivant la position et l'orientation des rayons,

e) filtrage des valeurs de liaison ($l(\theta_{i1}, t_1; \theta_{i2}, t_2)$) pour la production de données de filtre ($l(\theta_{i1}, t_1; \theta_{i2}, t_2)$) pour des lignes de liaison (51) qui sont affectées à des rayons qui traversent une surface déterminée (70) de la zone d'examen,

f) calcul de l'atténuation (f(x,y,z)) du rayonnement en pixels sur la surface (70) par addition des données de filtre ($l(\theta_{i1}, t_1; \theta_{i2}, t_2)$) de lignes de liaison qui approchent la trajectoire (30) définie dans l'espace de paramètres ($\theta$, $t$, $s$) par les rayons qui traversent le pixel respectif,

g) répétition des étapes e) et f) au moins pour d'autres surfaces (70) qui sont décalées l'une par rapport à l'autre dans la direction de l'axe de rotation.

**2.** Procédé de tomographie informatisée selon la revendication 1,

**caractérisé en ce**

**que** les surfaces (70) sont définies respectivement par un réseau de rayons (41,..., 45) qui sont situés dans des plans parallèles à l'axe de rotation (14) et relient entre eux les segments opposés de l'hélice (17).

**3.** Procédé de tomographie informatisée selon la revendication 1,

**caractérisé par** une pondération des valeurs de mesure avec le cosinus de l'angle que renferme le rayon appartenant respectivement à la valeur de mesure avec un plan perpendiculaire à l'axe de rotation.

**4.** Procédé de tomographie informatisée selon la revendication 1,

**caractérisé par** le calcul des valeurs d'atténuation (f(x,y,z)) sur les points de grille d'une grille tridimensionnelle régulière, de préférence d'une grille cubique, à partir des valeurs d'atténuation (f(x,y)) des pixels (x,y) sur les surfaces (70).

**5.** Procédé de tomographie informatisée selon la revendication 1,

des valeurs de mesure à partir d'une plage angulaire de $180°/N_1$ étant utilisées pour le calcul de chaque valeur de liaison, $N_1$ ayant, du moins approximativement, la valeur $\sqrt{N_\theta}$ et $N_\theta$ étant le nombre des positions de la source de rayonnement réparties dans une plage angulaire de $180°$ dans lesquelles les valeurs de mesure sont saisies.

**6.** Procédé de tomographie informatisée selon la revendication 1 avec un rebinning des valeurs de mesure acquises ($p(\beta,\gamma,s)$) tel qu'il en résulte un nouveau jeu de valeurs de mesure ($p^P(\theta,t,s)$) affectées à des rayons qui se trouvent sur une grille cartésienne dans un espace de paramètres ($\theta,t,s$) défini par la direction ($\theta$) des rayons dans un plan perpendiculaire à l'axe de rotation et par leurs positions ($t,s$) dans un plan (160) contenant l'axe de rotation (14).

**7.** Tomographe informatisé pour la mise en oeuvre du procédé selon la revendication 1,

- avec une source de rayons (S) pour la production d'un faisceau conique de rayons (4) qui traverse une zone d'examen (13) ou un objet qui s'y trouve,
- avec un dispositif d'entraînement (2,5) pour la production d'un mouvement relatif entre la source de rayons (S) et la zone d'examen sous la forme d'une hélice (17),
- avec une unité de détecteur (16) pour l'acquisition de valeurs de mesure qui dépendent de l'atténuation des rayons dans la zone d'examen;
- et avec une unité de reconstruction (10) pour la détermination de la distribution spatiale des valeurs d'atténuation (f,x,y,z) dans la zone d'examen **caractérisé en ce que** l'unité de reconstruction traite les valeurs de mesure selon la procédure suivante:

a) calcul des valeurs de liaison ($l(\theta_{i1}, t_1; \theta_{i2}, t_2)$) par addition des valeurs de mesure le long de lignes de liaison (51) d'un réseau (40) dans un espace de paramètres ($\theta$, $t$, s) tridimensionnel décrivant la position et l'orientation des rayons,

b) filtrage des valeurs de liaison ($I(\theta_{i1}, t_1; \theta_{i2}, t_2)$) pour la production de données de filtre (I($\theta_{i1}$, t$_1$; $\theta_{i2}$, t$_2$)) pour des lignes de liaison (51) qui sont affectées à des rayons qui traversent une surface déterminée (70) de la zone d'examen,

c) calcul de l'atténuation (f(x,y,z)) du rayonnement en pixels sur la surface (70) par addition des données de filtre (I($\theta_{i1}$, t$_1$; $\theta_{i2}$, t$_2$)) de lignes de liaison qui approchent la trajectoire (30) définie dans l'espace de paramètres ($\theta$, $t$, $s$) par les rayons qui traversent le pixel respectif,

d) répétition des étapes e) et f) au moins pour d'autres surfaces (70) qui sont décalées l'une par rapport à l'autre dans la direction de l'axe de rotation.

8. Programme informatique de traitement des valeurs de mesure d'un tomographe informatisé

- avec une source de rayons (S) pour la production d'un faisceau conique de rayons (4) qui traverse une zone d'examen (13) ou un objet qui s'y trouve,
- avec un dispositif d'entraînement (2,5) pour la production d'un mouvement relatif entre la source de rayons (S) et la zone d'examen sous la forme d'une hélice (17),
- avec une unité de détecteur (16) pour l'acquisition de valeurs de mesure qui dépendent de l'atténuation des rayons dans la zone d'examen;
- et avec une unité de reconstruction (10) pour la détermination de la distribution spatiale des valeurs d'atténuation (f,x,y,z) dans la zone d'examen, **caractérisé par** les étapes suivantes:

a) calcul des valeurs de liaison ($I(\theta_{i1}, t_1; \theta_{i2}, t_2)$) par addition des valeurs de mesure le long de lignes de liaison (51) d'un réseau (40) dans un espace de paramètres ($\theta$, $t$, s) tridimensionnel décrivant la position et l'orientation des rayons,

b) filtrage des valeurs de liaison ($I(\theta_{i1}, t_1; \theta_{i2}, t_2)$) pour la production de données de filtre (I($\theta_{i1}$, t$_1$; $\theta_{i2}$, t$_2$)) pour des lignes de liaison (51) qui sont affectées à des rayons qui traversent une surface déterminée (70) de la zone d'examen,

c) calcul de l'atténuation (f(x,y,z)) du rayonnement en pixels sur la surface (70) par addition des données de filtre (I($\theta_{i1}$, t$_1$; $\theta_{i2}$, t$_2$)) de lignes de liaison qui approchent la trajectoire (30) définie dans l'espace de paramètres ($\theta$, $t$, $s$) par les rayons qui traversent le pixel respectif,

d) répétition des étapes e) et f) au moins pour d'autres surfaces (70) qui sont décalées l'une par rapport à l'autre dans la direction de l'axe de rotation.

FIG. 1

FIG. 2

FIG. 3

FIG. 4

FIG. 5

FIG. 6

FIG. 7

FIG. 8

FIG. 9

FIG. 10